# EUROPEAN PATENT SPECIFICATION

(11) **EP 3 129 052 B1**
(45) Date of publication and mention of the grant of the patent: **03.06.2020**
(21) Application number: 15775944.0
(22) Date of filing: 07.04.2015
(51) Int. Cl.: A61K 39/395, C07K 16/28, A61K 47/42, A61P 35/02

(54) **OPTIMAL DOSING OF A CD19-ANTIBODY DRUG CONJUGATE**
OPTIMALE DOSIERUNG EINES CD19-ANTKÖRPER-ARZNEIMITTELKONJUGATS
DOSAGE OPTIMAL D'UN CONJUGUÉ MÉDICAMENT-ANTICORPS ANTI-CD19

(30) Priority: 08.04.2014 US 201461976790 P
(43) Date of publication of application: 15.02.2017
(73) Proprietor: Seattle Genetics, Inc., Bothell, WA 98021 (US)
(72) Inventor: ZHAO, Baiteng, Bothell, Washington 98021 (US); ALBERTSON, Tina, Bothell, Washington 98021 (US); HAN, Tae, Bothell, Washington 98021 (US)
(74) Representative: Gowshall, Jonathan Vallance
(86) International application number: PCT/US2015/024732
(87) International publication number: WO 2015/157297

(56) References cited:
- WO-A2-2012/162561
- WO-A2-2012/162561
- US-B2- 7 968 687
- US-B2- 7 968 687
- Uma Borate et al.: "A First-In-Human Phase 1 Study Of The Antibody-Drug Conjugate SGN-CD19A In Relapsed Or Refractory B-Lineage Acute Leukemia and Highly Aggressive Lymphoma", BLOOD, vol. 122, no. 21, 1437, 15 November 2013 (2013-11-15), XP002774264, Retrieved from the Internet: URL:http://www.bloodjournal.org/content/12 2/21/1437
- HOBBS ROBERT F ET AL: "Radiobiologic optimization of combination radiopharmaceutical therapy applied to myeloablative treatment of non-Hodgkin lymphoma.", JOURNAL OF NUCLEAR MEDICINE : OFFICIAL PUBLICATION, SOCIETY OF NUCLEAR MEDICINE SEP 2013, vol. 54, no. 9, September 2013 (2013-09), pages 1535-1542, XP002774265, ISSN: 1535-5667
- BORATE ET AL.: 'A First-In-Human Phase 1 Study Of The Antibody-Drug Conjugate SGN- CD 19A In Relapsed Or Refractory B-Lineage Acute Leukemia and Highly Aggressive Lymphoma' BLOOD vol. 122, no. 21, 15 November 2013, page 1437

## Description

### FIELD OF THE INVENTION

This invention relates to optimal dose regimens for treatment of B-linage acute lymphoblastic leukemia (B-ALL or ALL) using a CD19-antibody drug conjugate.

### BACKGROUND OF THE INVENTION

CD19 is a member of the immunoglobulin superfamily. *See*, *e.g.,* Tedder & Isaacs, J Immunol, 143:712-717 (1989) and Del Nagro et al., Immunol Res, 31:119-131 (2005). It is a B cell-specific marker not known to be expressed by any cell outside of the B lineage. CD19 expression is maintained upon malignant transformation, thus, CD19 is found on malignant cells in the majority of patients with B-cell leukemia or non-Hodgkin lymphoma. *See, e.g.,* Nadler et al., J Immunol, 131:244-250 (1983); Anderson et al., Blood, 63:1424-1433 (1984); and Scheuermann & Racila, Leuk Lymphoma, 18:385-397 (1995).

SGN-CD19A is a CD19-directed antibody-drug conjugate (ADC) consisting of three components: 1) the humanized antibody hBU12, specific for human CD19, 2) the microtubule disrupting agent, monomethyl auristatin F (MMAF), and 3) a stable linker, maleimidocaproyl, that covalently attaches MMAF to hBU12. The proposed mechanism of action (MOA) is initiated by SGN-CD19A binding to CD19 on the cell surface followed by internalization of the ADC. Upon trafficking to lysosomes, the delivered drug (cysmcMMAF) is released through proteolytic degradation of the antibody carrier. Binding of the released drug to tubulin disrupts the microtubule network, leading to cell cycle arrest and apoptosis.

SGN-CD19A activity has recently been assessed in a phase 1 clinical trial for treatment of patients with B-linage acute lymphoblastic leukemia (B-ALL). The trial is intended to provide information on the safety of the SGN-CD19A molecule in a human patient population. Clinical trials also provide data on the pharmacokinetics and efficacy of the SGN-CD19A molecule in treating B-ALL. For best usage of the drug, an optimum dosing regimen would be useful for prescribing physicians. The present invention solves this and other problems.

Borate et al., 2013 discloses the medical use of SGN-CD19A, which is the anti CD19 humanised antibody BU12 conjugated to the microtubule - disrupting agent monomethyl auristatin F (MMAF), tested in a Phase I study in acute leuikemia and lymphoma.

WO 2012/162561 discloses the use of CD19-ADC for treating acute lymphoblastic leukemia.

US 7 968 687 B2 discloses the preparation of CD19 binding agents and their use in treating disease.

### BRIEF SUMMARY OF THE INVENTION

The invention is defined by the claims.

The present invention provides a method for use in treating acute lymphoblastic leukemia (ALL) by first administering a loading dose of a CD19-antibody drug conjugate, wherein the CD19-antibody drug conjugate (CD19-ADC) is a humanized BU12 antibody conjugated to an mcMMAF molecule, followed by administering a maintenance dose of the CD19-antibody drug conjugate, the maintenance dose being 2.0 mg/kg body weight every three weeks. The humanized hBU12 antibody has a light chain variable region of SEQ ID NO:1 and a heavy chain variable region of SEQ ID NO:2. In some embodiments, the subject has a high disease burden and is sensitive to treatment with the CD19 ADC; the loading dose for such subject is 2.0 mg/kg body weight and is administered on days one and eight of a three week cycle. The loading dose can be administered in a single three week cycle or two three or four loading doses can be administered before a maintenance dose is started. The 2 mg/kg maintenance dose is administered on day one of a three week cycle for multiple three week cycles. In some embodiments, maintenance doses are given for between one and ten three week cycles. In further embodiments, maintenance doses are given for between ten and twenty three week cycles. In preferred embodiments, the administration of the CD19 ADC is intravenous administration. In further embodiments, the CD19 ADC maintenance dose is administered at a dose required to maintain 90% occupancy of the CD19 receptor on blast cells.

In one embodiment, the disclosure provides a method of treating a subject with ALL that has a high disease burden and is insensitive to treatment with the CD19 ADC using a loading dose followed by a maintenance dose. Such a subject is treated with a 5 mg/kg loading dose of the CD19-ADC that is a humanized BU12 antibody conjugated to an mcMMAF molecule, which is administered on days one and eight of a three week cycle. The loading dose can be administered in a single three week cycle or two three or four loading doses can be administered before a maintenance dose is started. The maintenance dose is 3 mg/kg and is administered on day one of a three week cycle for multiple three week cycles. In some embodiments, maintenance doses are given for between one and ten three week cycles. In further embodiments, maintenance doses are given for between ten and twenty three week cycles. In preferred embodiments, the administration of the CD19 ADC is intravenous administration. In further embodiments, the CD19 ADC maintenance dose is administered at a dose required to maintain 90% occupancy of the CD 19 receptor on blast cells.

In one embodiment, the disclosure provides a method of treating a subject having acute lymphoblastic leukemia (ALL) by administering a fixed dose of a CD19-antibody drug conjugate, wherein the CD19-antibody drug conjugate (CD19-ADC) is a humanized BU12 antibody conjugated to an mcMMAF molecule, wherein the dose intensity is about 1 mg/kg/week. The humanized hBU12 antibody has a light chain variable region of SEQ ID NO:1 and a heavy chain variable region of SEQ ID NO:2. In one embodiment, the fixed dose is 1.6 mg/kg body weight and is administered on days one and seven of a three week cycle. In another embodiment, the fixed dose is 2.2 mg/kg body weight and is administered every two weeks. In a further embodiment, the fixed dose is 3.0 mg/kg body weight and is administered every three weeks.. In some embodiments, fixed doses are given for between one and ten cycles. In further embodiments, fixed doses are given for between ten and twenty cycles. In additional embodiments, fixed doses are administered for up to 50 cycles. In preferred embodiments, the administration of the CD19 ADC is intravenous administration. In further embodiments, the CD19 ADC maintenance dose is administered at a dose required to maintain 90% occupancy of the CD19 receptor on blast cells.

### DEFINITIONS

The term "CD 19" refers to "cluster of differentiation protein 19", a human protein that is expressed on human B cells. The amino acid sequence of human CD19 is known and is disclosed, e.g., at NCBI Reference Sequence: NP_001171569.1.

A "disorder", as used herein, and the terms "CD19-associated disorder" and "CD19-associated disease" refer to any condition that would benefit from treatment with a CD19-antibody drug conjugate (CD19-ADC), such as SGN-CD19A, as described herein. This includes chronic and acute disorders or diseases including those pathological conditions that predispose the mammal to the disorder in question. Non-limiting examples or disorders to be treated herein include CD19 expressing cancers, including hematological malignancies, benign and malignant tumors, leukemias and lymphoid malignancies, as well as inflammatory, angiogenic and immunologic disorders. Specific examples of disorders are disclosed *infra.*

In certain aspects of the present disclosure, B cell malignancies, also referred to as B-cell lineage malignancies, are treatable by the methods of the present invention. The term B cell malignancies include any malignancy that is derived from a cell of the B cell lineage.

The terms "treatment" and "therapy", and the like, as used herein, are meant to include therapeutic or suppressive measures for a disease or disorder leading to any clinically desirable or beneficial effect, including, but not limited to, alleviation or relief of one or more symptoms, regression, slowing or cessation of progression of the disease or disorder. For example, treatment can include a decrease or elimination of a clinical or diagnostic symptom of a CD 19-expressing disorder after the onset of the clinical or diagnostic symptom by administration of an anti-CD19 antibody or other CD19 binding agent to a subject. Treatment can be evidenced as a decrease in the severity of a symptom, the number of symptoms, or frequency of relapse.

The terms "subject" or "patient" may be used interchangeably and refer to mammals such as human patients and non-human primates, as well as experimental animals such as rabbits, dogs, cats, rats, mice, and other animals. Accordingly, the term "subject" or "patient" as used herein means any mammalian patient or subject to which the CD19 binding agents of the invention can be administered. In preferred embodiments, the terms subject or patient are used to refer to human patients. In certain aspects of the present disclsoure, subjects may include those that have been diagnosed with a CD19 expressing cancer, including, for example, B cell lymphoma or B cell leukemia, including, non-Hodgkin lymphoma, chronic lymphocytic leukemia, and acute lymphoblastic leukemia. In certain aspects of the present disclosure, the subject will have a refractory or relapsed CD19 expressing cancer

A subject with a refractory CD19 expressing cancer is a subject who does not respond to therapy, *i.e.,* the subject continues to experience disease progression despite therapy.

A subject with a relapsed CD19 expressing cancer is a subject who has responded to the therapy at one point, but has had a recurrence or further progression of disease following the response.

The term "effective amount" refers to the amount of a CD19-ADC, e.g., SGN-CD19A, that is sufficient to inhibit the occurrence or ameliorate one or more clinical or diagnostic symptoms of a CD19-associated disorder in a subject. An effective amount of an agent is administered according to the methods described herein in an "effective regimen." The term "effective regimen" refers to a combination of amount of the agent and dosage frequency adequate to maintain high CD 19 occupancy, which may accomplish treatment or prevention of a CD19-associated disorder. In a preferred embodiment, an effective regimen maintains near complete, e.g., greater than 90%, CD19 occupancy on CD19-expressing cells during dosing intervals.

The term "pharmaceutically acceptable" as used herein refers to those compounds, materials, compositions, and/or dosage forms that are, within the scope of sound medical judgment, suitable for contact with the tissues of human beings and animals without excessive toxicity, irritation, allergic response, or other problems or complications commensurate with a reasonable benefit/risk ratio. The term "pharmaceutically compatible ingredient" refers to a pharmaceutically acceptable diluent, adjuvant, excipient, or vehicle with which a CD19-ADC, e.g., SGN-CD19A is administered.

The term "pharmaceutically compatible ingredient" refers to a pharmaceutically acceptable diluent, adjuvant, excipient, or vehicle with which a CD19-ADC, e.g., SGN-CD19A, is administered.

As used herein, the term "about" denotes an approximate range of plus or minus 10% from a specified value. For instance, the language "about 20%" encompasses a range of 18-22%. As used herein, about also includes the exact amount. Hence "about 20%" means "about 20%" and also "20%."

### BRIEF DESCRIPTION OF THE DRAWINGS

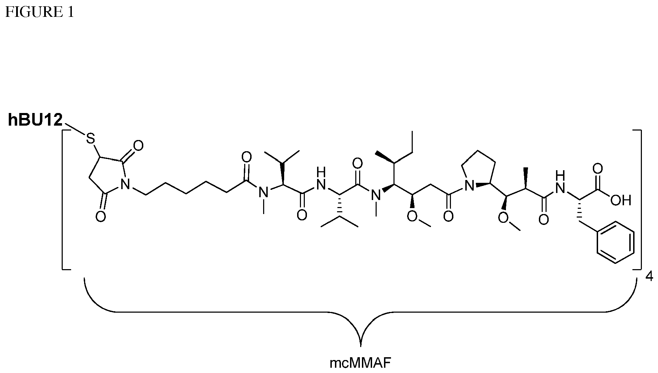

Figure 1 provides the structure of SGN-CD19A. The humanized antibody hBU12 is conjugated to the mcMMAF drug linker.

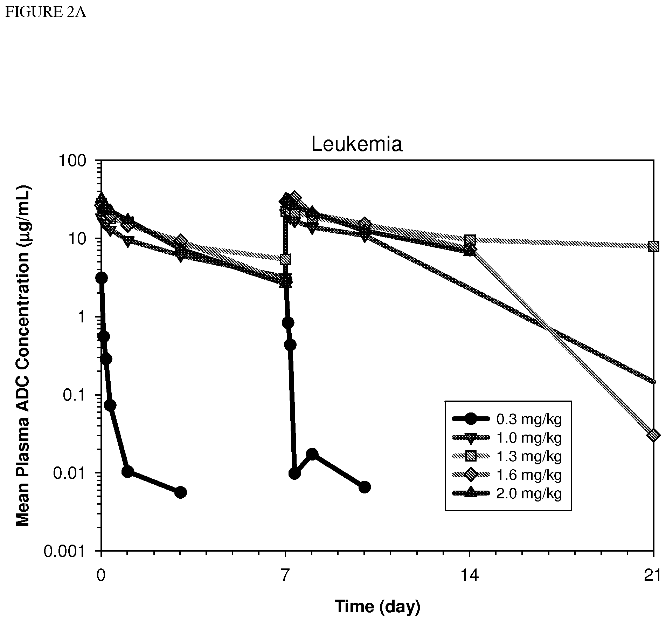

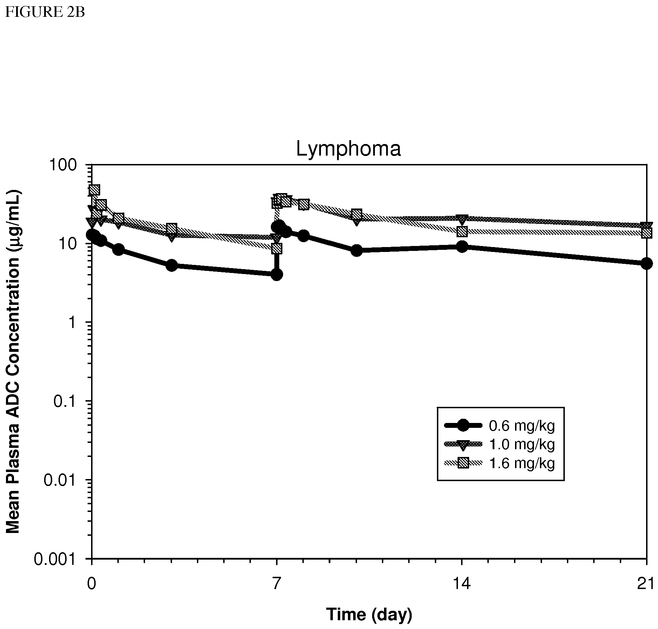

Figures 2A and 2B provide mean pharmacokinetic profiles of CD19-ADC from leukemia (Figure 2A) and lymphoma (Figure 2B) patients in Cycle 1 of the treatment. Doses of the CD19-ADC were administered to patients and the plasma CD19-ADC concentrations were measured. The mean plasma CD19-ADC concentrations at each dose level were shown over time. The patients had either acute lymphoblastic leukemia (B-ALL) or highly aggressive lymphoma. Patients received the indicated doses of the CD19-ADC on a weekly schedule.

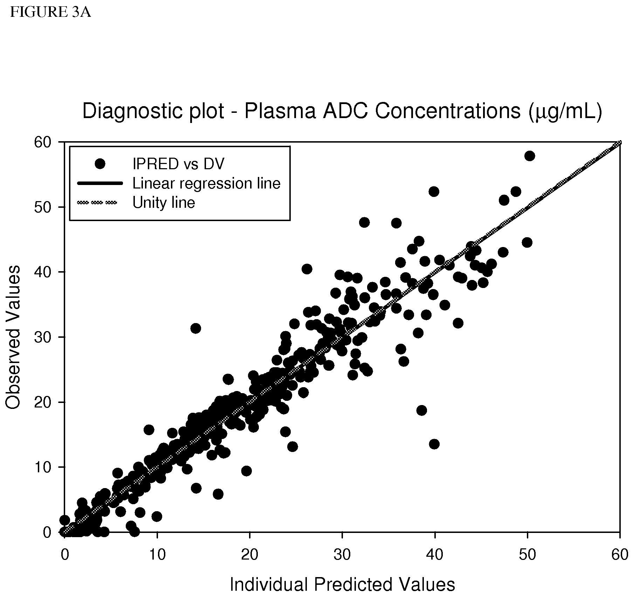

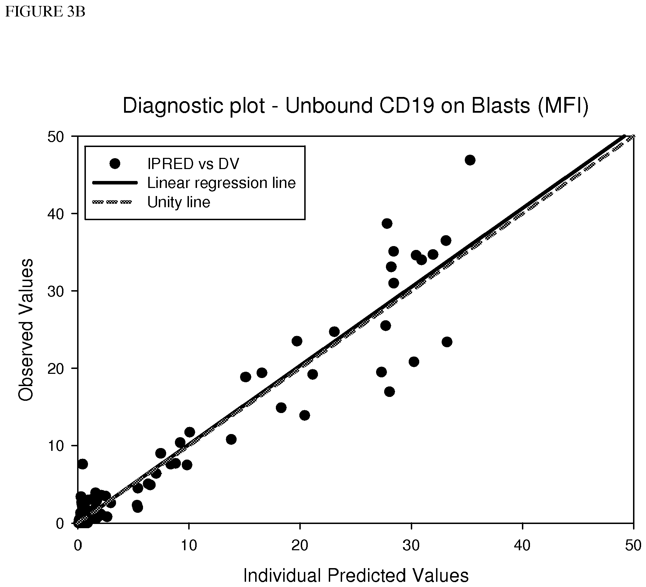

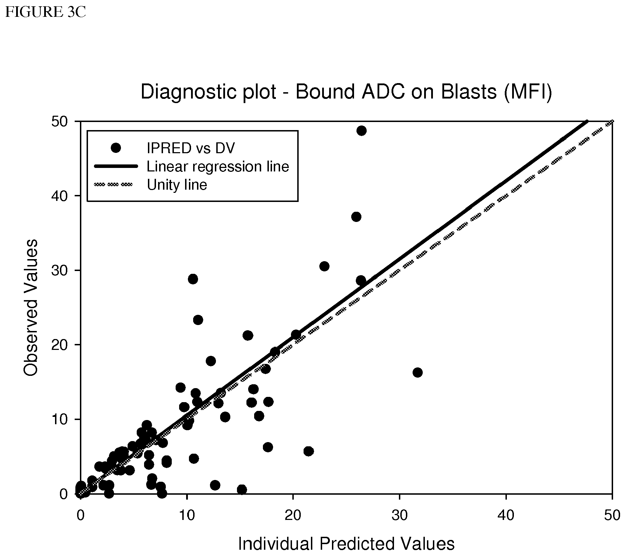

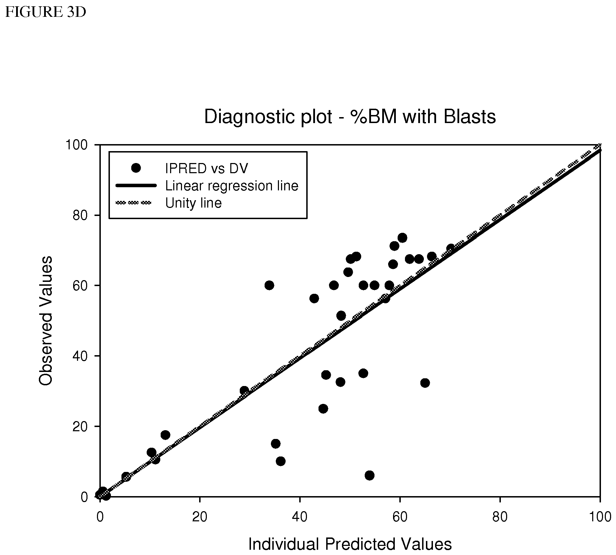

Figures 3A-3D provide diagnostics of the model fitting by comparison of observed values versus values predicted by the model for plasma ADC concentrations (Figure 3A), unbound CD 19 mean fluorescent intensity (MFI) on blasts (Figure 3B), bound ADC MFI on blasts (Figure 3C), and percent bone marrow with blasts (Figure 3D). The linear regression lines are solid and the unity line is a dashed line.

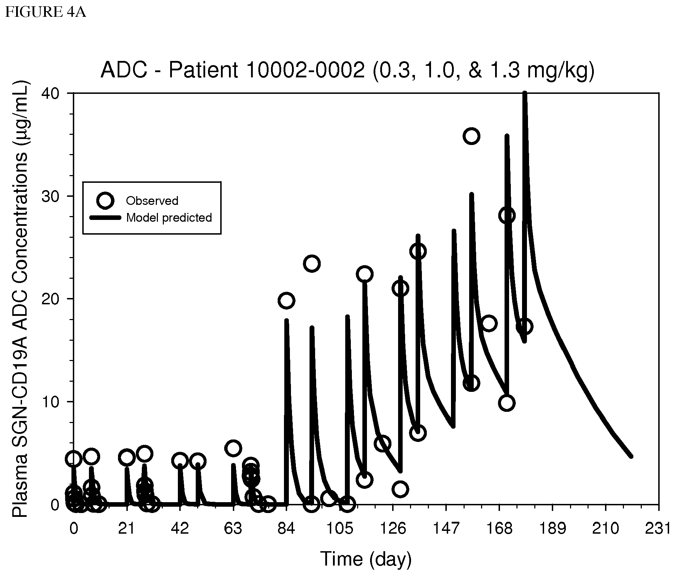

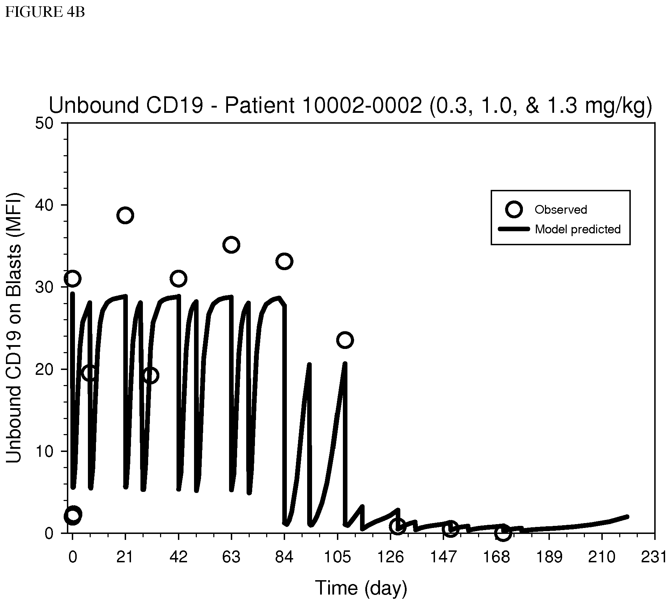

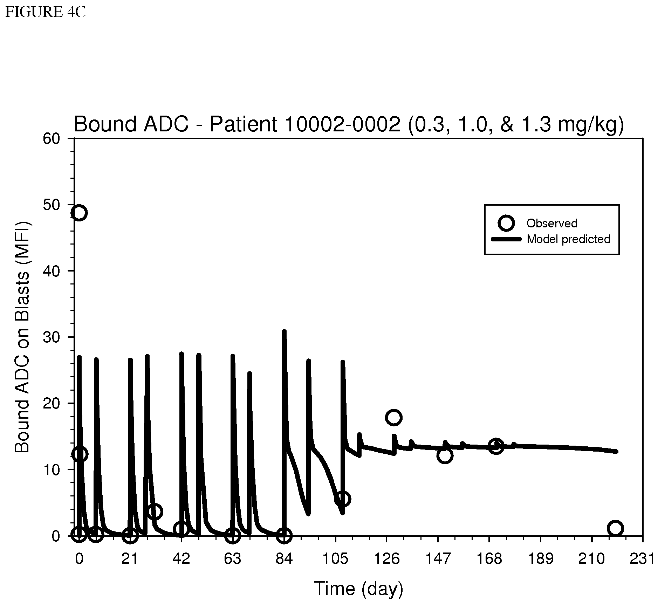

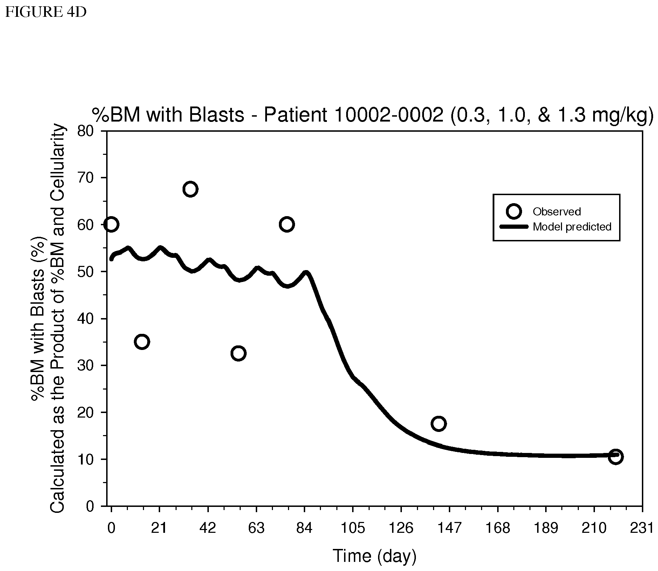

Figures 4A-4D provide a case study of individual fit diagnostics for an ALL patient. Actual and predicted values are provided for plasma CD19-ADC concentrations (figure 4A), unbound CD19 MFI on blast cells (Figure 4B), bound CD19-ADC MFI to blast cells (Figure 4C), and percentage of bone marrow with blasts (Figure 4D).

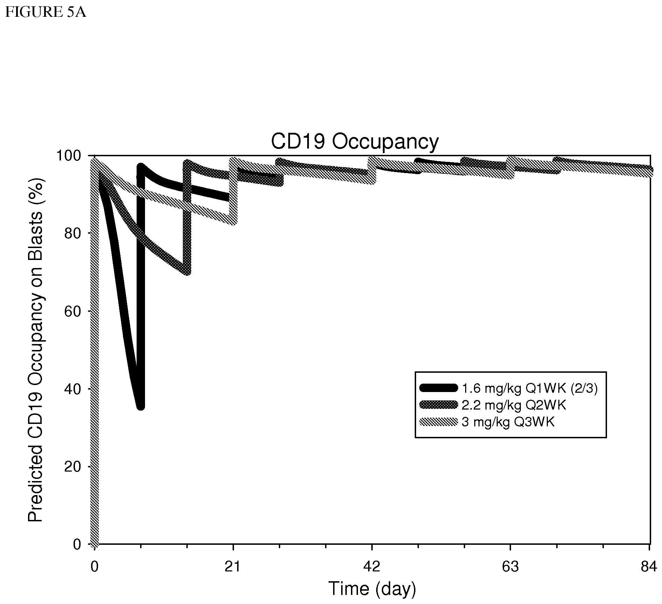

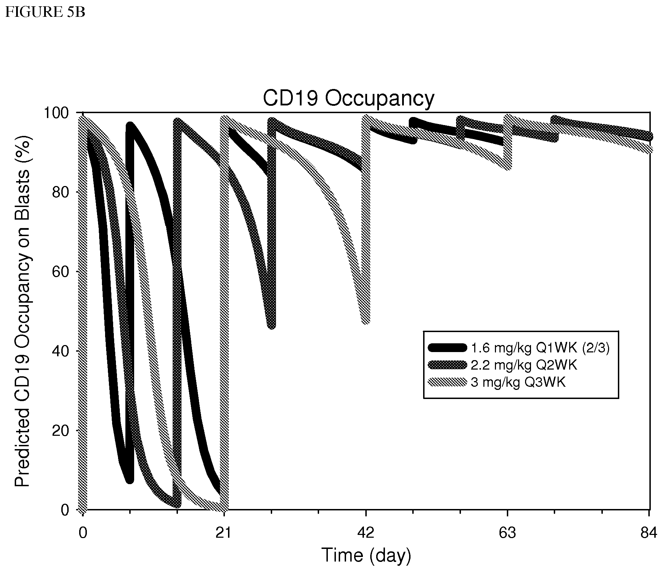

Figures 5A and 5B provide CD19 occupancy predicted by the model for a fixed dosage schedule of CD19-ADC treatment of ALL. The simulated scenario included high baseline CD19 expression and 100% bone marrow blasts and hypercellularity. Figure 5A provides the results for cancer that is sensitive to treatment, while Figure 5B provides the results for a cancer that is less sensitive to treatment.

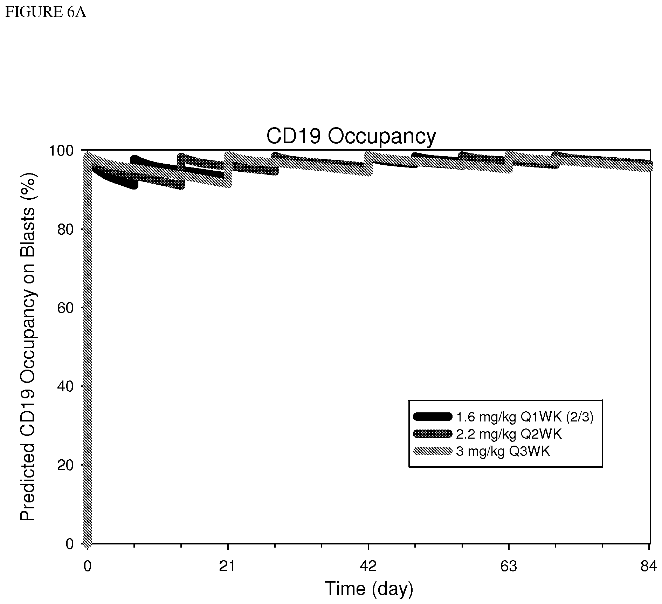

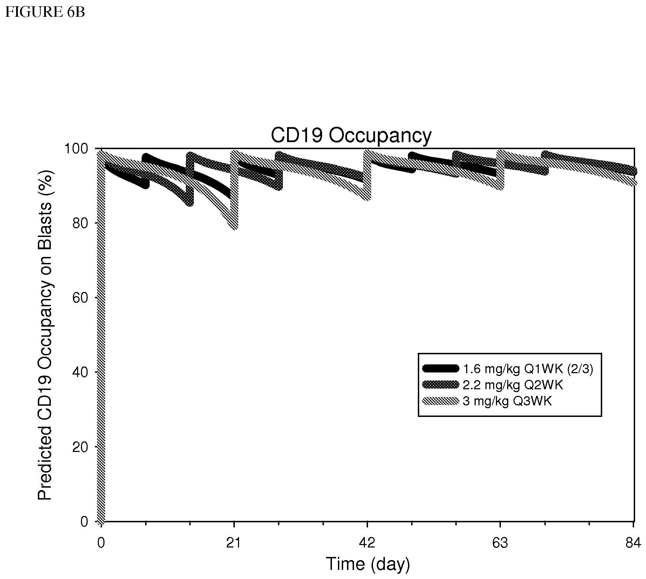

Figures 6A and 6B provide CD19 occupancy predicted by the model for a fixed dosage schedule of CD19-ADC treatment of ALL. The simulated scenario included high baseline CD19 expression and 10% bone marrow blasts and blast hypercellularity. Figure 6A provides the results for cancer that is sensitive to treatment, while Figure 6B provides the results for a cancer that is less sensitive to treatment.

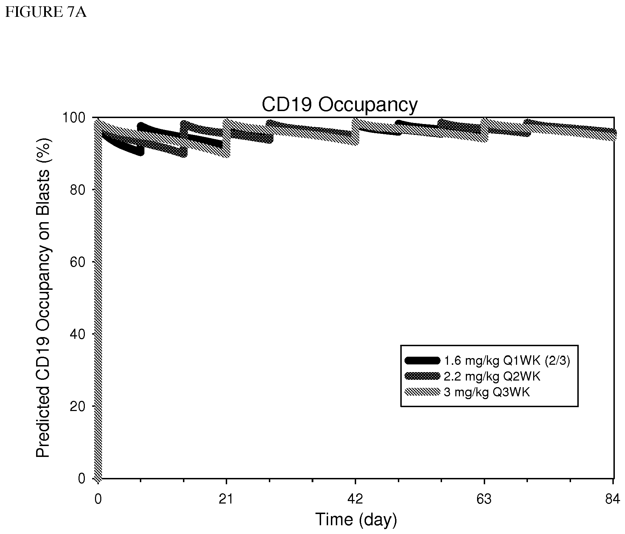

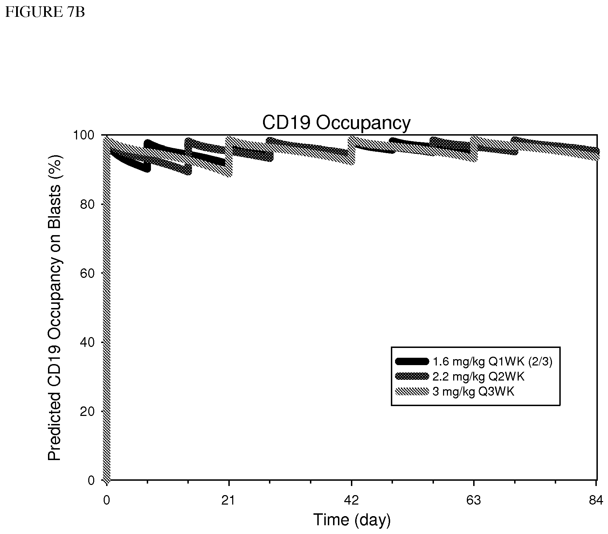

Figures 7A and 7B provide CD19 occupancy predicted by the model for a fixed dosage schedule of CD19-ADC treatment of ALL. The simulated scenario included low baseline CD19 expression and 100% bone marrow blasts and blast hypercellularity. Figure 7A provides the results for cancer that is sensitive to treatment, while Figure 7B provides the results for a cancer that is less sensitive to treatment.

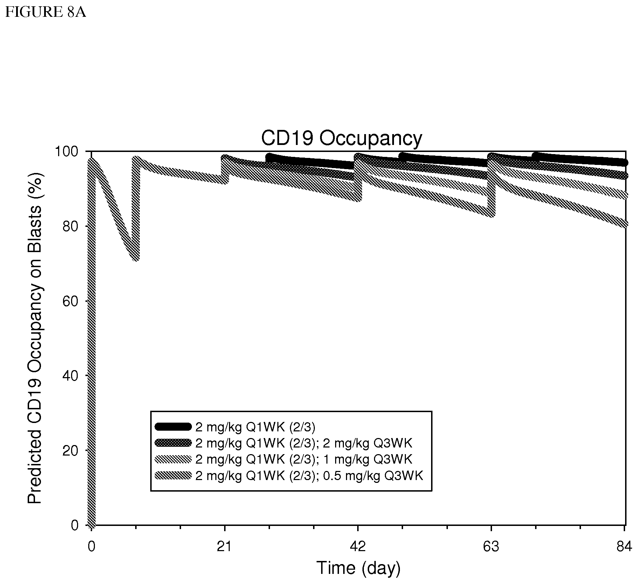

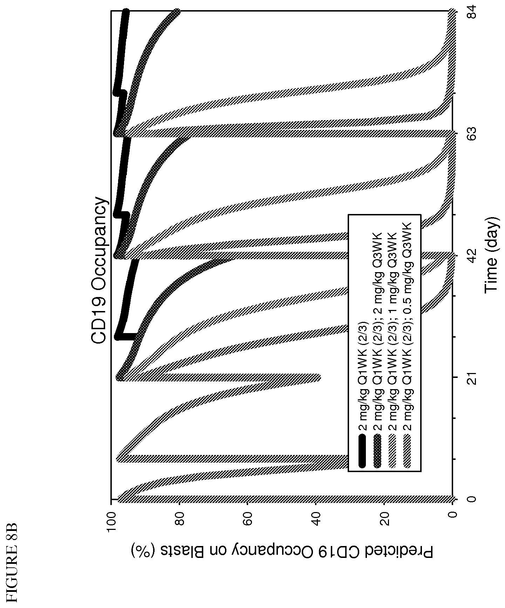

Figures 8A and 8B provide CD19 occupancy predicted by the model for a more frequent dosage of CD19-ADC, followed by less frequent maintenance dosage of CD19-ADC for treatment of ALL. The simulated scenario included high baseline CD 19 expression and 100% bone marrow blasts and hypercellularity. The initial cycle was 2 mg/kg given weekly on days 1 and 8; varied maintenance doses were given every three weeks. Figure 8A provides the results for cancer that is sensitive to treatment, while Figure 8B provides the results for a cancer that is less sensitive to treatment.

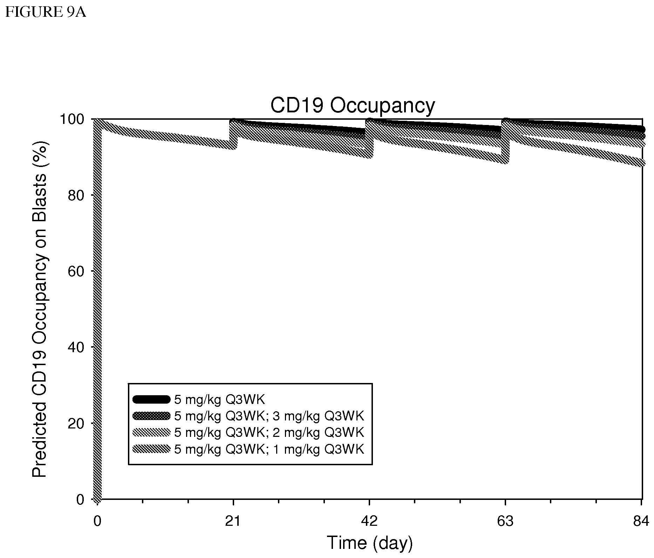

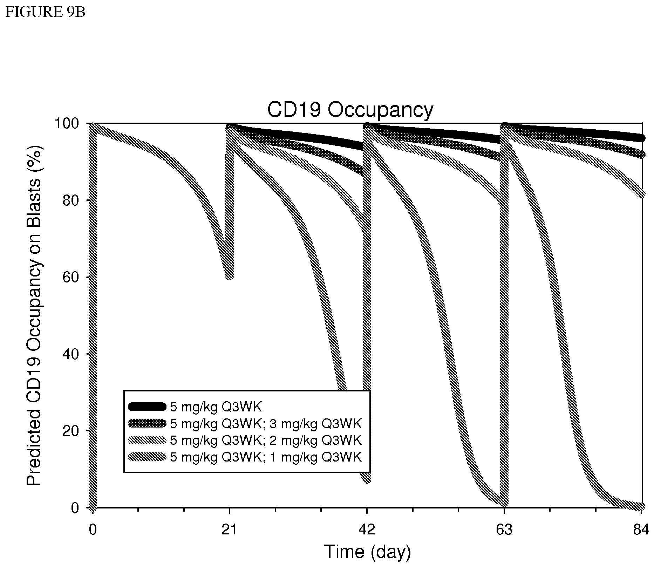

Figures 9A and 9B provide CD19 occupancy predicted by the model for a loading dose of CD19-ADC, followed by maintenance dosage of CD19-ADC for treatment of ALL. The simulated scenario included high baseline CD 19 expression and 100% bone marrow blasts and hypercellularity. The loading dose was 5 mg/kg; maintenance doses were varied. Figure 9A provides the results for cancer that is sensitive to treatment, while Figure 9B provides the results for a cancer that is less sensitive to treatment.

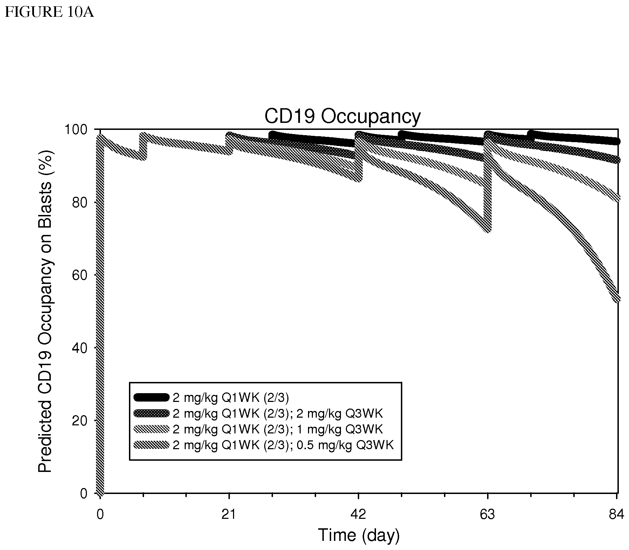

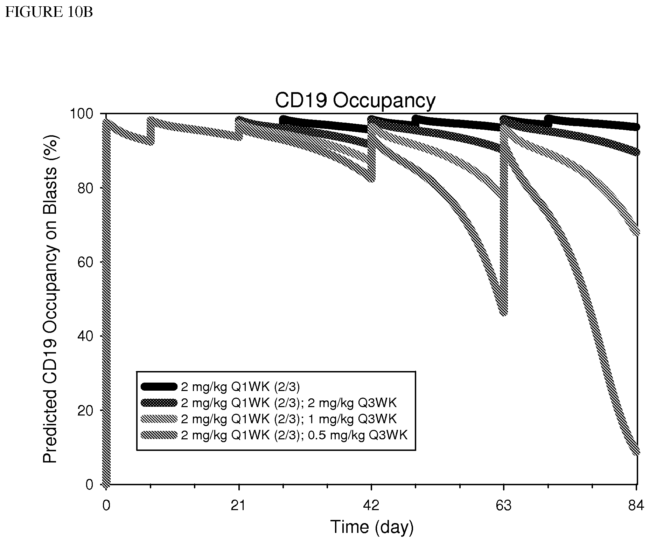

Figures 10A and 10B provide CD19 occupancy predicted by the model for a more frequent dosage of CD19-ADC, followed by less frequent maintenance dosage of CD19-ADC for treatment of ALL. The simulated scenario included low baseline CD19 expression and 100% bone marrow blasts and hypercellularity.. The initial cycle was 2 mg/kg given weekly on days 1 and 8; varied maintenance doses were given every three weeks. Figure 10A provides the results for cancer that is sensitive to treatment, while Figure 10B provides the results for a cancer that is less sensitive to treatment.

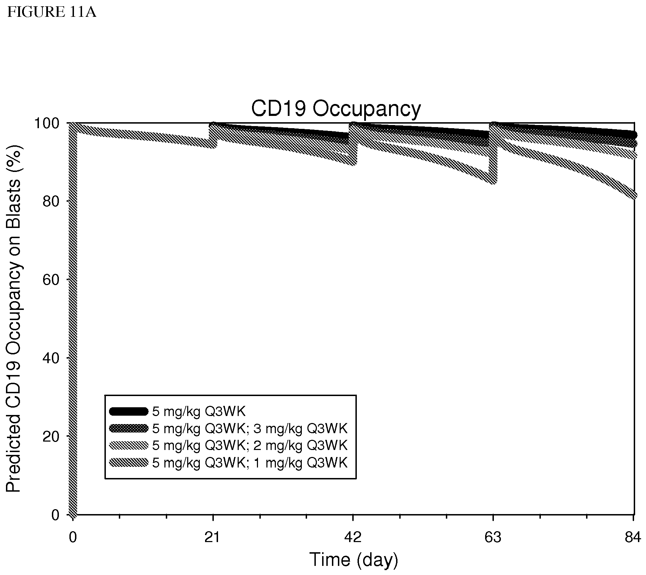

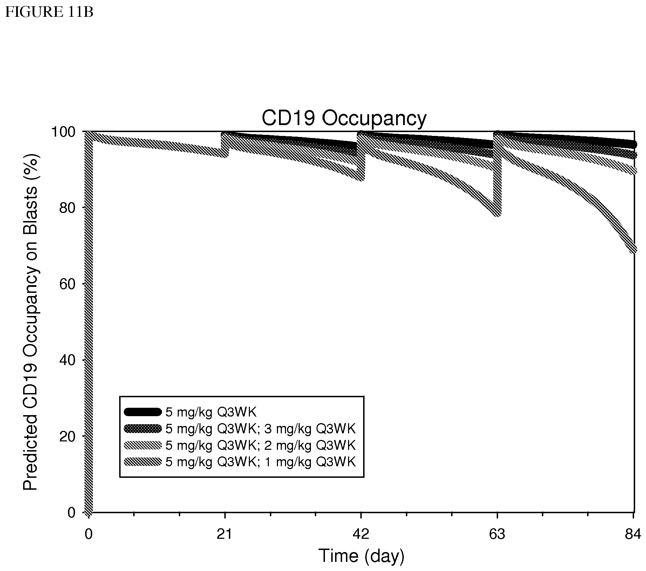

Figures 11A and 11B provide CD19 occupancy predicted by the model for a loading dose of CD19-ADC, followed by maintenance dosage of CD19-ADC for treatment of ALL. The simulated scenario included low baseline CD19 expression and 100% bone marrow blasts and hypercellularity. The loading dose was 5 mg/kg every three weeks; maintenance doses were varied. Figure 11A provides the results for cancer that is sensitive to treatment, while Figure 11B provides the results for a cancer that is less sensitive to treatment.

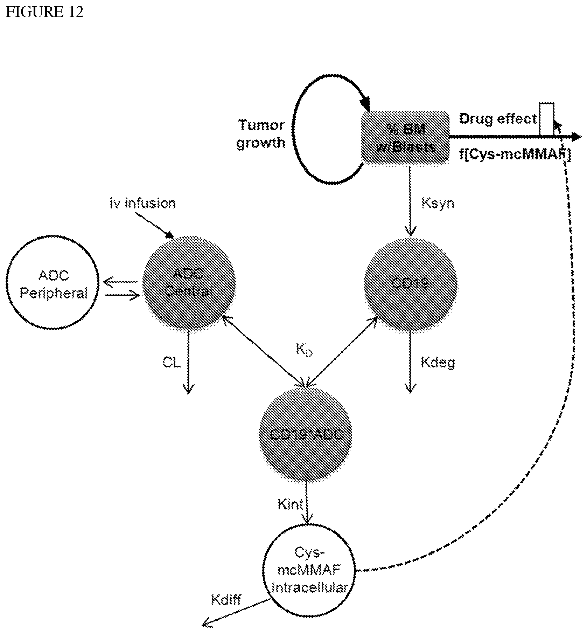

Figure 12 provides the model structure.

### DETAILED DESCRIPTION

The present disclosure provides optimized dosage schedules for administration of the SGN-CD19A drug to patients suffering from acute lymphoblastic leukemia (ALL). Results from an early stage clinical trial indicate that SGN-CD19A exhibits a complex relationship between pharmacokinetics (PK) and pharmacodynamics (PD). A model was generated to describe the relationship of SGN-CD19A clearance and its effect on ALL tumor cells. Using the model, SGN-CD19A dosing schedules can be devised to maximize tumor cell cytotoxicity and minimize off target toxicity in the patient.

SGN-CD19A is an antibody drug conjugate (ADC) produced by the conjugation of the drug-linker intermediate maleimidocaproyl monomethyl auristatin F (mcMMAF) to the humanized antibody hBU12 (Figure 1). The points of attachment are cysteines produced by reduction of inter-chain disulfides. SGN CD19A has an average of four drugs per antibody molecule.

Methods of making the hBU12 antibody are disclosed, e.g., at US Patent No. 7,968,687. The amino acid sequence of the light chain variable region of hBU12 is provided herein as SEQ ID NO:1. The amino acid sequence of the heavy chain variable region of hBU12 is provided herein as SEQ ID NO:2. hBU12 is an IgG1 antibody and the variable regions are joined to human heavy and light constant regions. US Patent No. 7,968,687 also provides methods for the synthesis of mcMMAF and its conjugation to hBU12.

SGN-CD19A, therefore, is an ADC that delivers mcMMAF to CD19-positive cells. mcMMAF is a tubulin-binding molecule. SGN-CD19A has a proposed multi-step mechanism of action initiated by binding to their target on the cell surface and subsequent internalization. After cell surface binding, internalization, and trafficking of SGN-CD19A through the endocytic pathway, proteolytic degradation of hBU12 in the lysosomes releases the cysteine adduct of the drug linker in the form of cys-mcMMAF, which then becomes available for tubulin binding. *See, e.g.,* Doronina et al., Nat Biotechnol 21:778-84 (2003) and Doronina et al., Bioconjug Chem 17: 114-24 (2006). cys-mcMMAF and mcMMAF are used interchangeably herein. Binding of the released drug to tubulin disrupts the cellular microtubule network, leading to G2/M phase cell cycle arrest and subsequent onset of apoptosis in the targeted cell.

The model described herein is used to optimize dosing of SGN-CD19A for treatment of acute lymphoblastic leukemia (ALL). ALL is also known as B-lineage acute lymphoblastic leukemia (B-ALL) and both names are used interchangeably. ALL is a cancer of the white blood cells that is characterized by uncontrolled growth of lymphoblasts or immature white blood cells. Lymphoblasts are also referred to as "blasts", herein. Blasts typically grow in the bone marrow and crowd out normal blood cells. Blasts cells may be seen in blood, but diagnoses is typically done be review of bone marrow biopsy.

Based on the PK and PD values from patients after administration of SGN-CD19A, a model was generated to describe the observed PK and PD data, and subsequently to determine optimal dosage of SGN-CD19A based on CD19 occupancy. See, e.g., Figure 12. According to the model, SGN-CD19A is first administered via intravenous infusion to the central compartment. The central compartment includes plasma, bone marrow, and rapidly perfused tissues. SGN-CD19A is then cleared from the central compartment at the rate of "CL" and may also diffuse to and from the more slowly perfused tissues, peripheral compartment, both through linear kinetics. SGN-CD19A in the central compartment binds to CD19 expressed on cancer cells, e.g., leukemia or lymphoma cells, to form a CD19-SGN-CD19A complex. The complex is internalized into the cancer cells, where the cytotoxin, cys-mcMMAF, is released after catabolism of SGN-CD19A. The intracellular cys-mcMMAF causes cancer cell growth arrest and death in a concentration-dependent manner. It is assumed that the majority of leukemia blast cells resides in bone marrow and replicate at a rate governed by the Gompertz growth law, which is faster at low disease burden and becomes slower with increasing disease burden. It is also assumed that all leukemia blast cells in a given patient have the same CD19 expression level and the total CD 19 in a patient is determined by the cellular expression level and disease burden at any given time.

Some assumptions underlie the model. First, the distribution kinetics of unbound SGN-CD19A (drug) to tissues is linear and may be described by inter-compartment rate constants. Second, SGN-CD19A is highly specific and does not bind to targets other than CD19. Third, the drug-CD 19 binding occurs only in the central compartment and not in the peripheral (tissue) compartments. Fourth, the drug-CD 19 binding is a rapid process and results in simple one-to-one drug-target complex. Fifth, the binding of SGN-CD19A to the CD19 protein results in internalization and degradation of the drug-CD 19 complex, which results in the released of the small molecule cytotoxin (cys-mcMMAF). Sixth, CD19 synthesis and degradation rates are constant in all blast cells in a given patient and are not affected by the treatment. Seventh, the majority of blast cells reside in bone marrow space.

The model structure is shown in Figure 12.

SGN-CD19A is formulated for patients in a sterile pharmaceutically acceptable excipient and then administered to patients. Typically, administration is by intravenous methods. A preferred pharmaceutically acceptable formulation for SGN-CD19A is 15 mg/ml in 10 mM potassium phosphate, pH 6.0, 0.02% w/v polysorbate 80, and 60 mg/ml sucrose.

The optimal doses and schedules of SGN-CD19A for treatment of ALL were determined based on a number of patient disease characteristics, for example, disease burden and CD19 expression levels. High disease burden in this model was defined as a packed (100% replaced), hypercellular bone marrow as assessed on bone marrow biopsy or aspirate. Low disease burden was defined as 10% of the bone marrow replaced by blasts as assessed on bone marrow biopsy or aspirate. High CD19 expression was defined as a CD19 MFI value of 45 at baseline and low expression was 4.5 at baseline. Another relevant characteristic is the sensitivity (coefficient of 0.00019 on drug effect) or lack of sensitivity (coefficient of 0.00006) of the cancer to an anti-CD 19 treatment, such as SGN-CD19A.

SGN-CD19A can be administered to ALL patients on a fixed dose schedule. A fixed dose schedule is a schedule that is unvaried over time. For SGN-CD19A administration on a fixed dose schedule, it is important to maintain the dose intensity, i.e., the amount of SGN-CD19A per unit of patient weight per unit of time, at about 1 mg/kg/week. This dose intensity will be beneficial to patients regardless of their disease burden or sensitivity to treatment. Thus, acceptable fixed dose schedules of SGN-CD19A include 1.6 mg/kg given on days one and eight of a three week cycle, 2.2 mg/kg every two weeks, or 3.0 mg/kg every three weeks.

SGN-CD19A can also be administered to ALL patients using a higher loading dose and/or more frequent loading doses followed by lower and/or less frequent maintenance doses. Typically, the loading dose is greater than the maintenance dose. For patients who have high disease burden and are sensitive to treatment, 2 mg/kg given every three weeks is an acceptable maintenance dose following one cycle of 2 mg/kg given on days one and eight of a three week cycle. Typically a single cycle of loading dose of more frequent dosing is given, but more can be administered, as needed, to decrease disease burden and induce remission before switching to maintenance dose schedule. Thus, one, two three or four loading doses can be given before starting maintenance doses. Maintenance doses are given, e.g., for two-20 three week cycles, at the 2 mg/kg dose level.

For patients who have high disease burden and are less sensitive to treatment, 3 mg/kg given every three weeks is an acceptable maintenance dose following a loading dose of 5 mg/kg given every three weeks. Typically a single loading dose is given, but more can be administered, as needed, to decrease disease burden and induce remission. For some patients, multiple loading doses, e.g., one, two three or four loading doses loading doses can be given before starting maintenance doses. Maintenance doses are given, e.g., for two-20 three week cycles, at the 3 mg/kg dose level.

### EXAMPLES

The following examples are offered to illustrate, but not to limit the claimed invention.

### Example 1: Model of SGN-CD19A dosing for treatment of ALL based on a semi-mechanistic PK/PD model.

### Methods

### Clinical trial

SGN-CD19A was administered intravenously (IV) on a weekly schedule (first 2 weeks of a 21-day cycle on Days 1 and 8. Dose levels were escalated. Adult patients were initially enrolled in cohorts of 2, starting with dose level 1. For the first 6 patients, complete dose limiting toxicity information was available before the next patient cohort was enrolled. After the first 6 patients, 3 patients at a time could be enrolled and in the DLT period. Once one patient finished the DLT period, another patient could be enrolled. Estimates of safety were determined by a continuous reassessment model, and an algorithm allocated patients to dose levels. Dose levels are shown in Table 1.

**Table 1: Dose escalation schedule**

| Dose Level | Schedule A: Weekly Dose (mg/kg) | Schedule B: Q3 week Dose (mg/kg) |
|---|---|---|
| 1 | 0.3 | 0.5 |
| 2 | 0.6 | 1 |
| 3 | 1 | 2 |
| 4 | 1.3 | 3 |
| 5 | 1.6 | 4 |
| 6 | 2 | 5 |
| 7 | 2.3 | 6 |
| 8 | 3 | - |
| 9 | 3.8 | - |
| 10 | 4.5 | - |

### PK and PD measurements

Plasma SGN-CD19A levels - SGN-CD19A was administered on days 1 and 8 of a 21 day cycle at the indicated dose levels. Blood was drawn from patients at the schedule time for PK and PD measurements (Table 2). The concentrations of plasma SGN-CD19A were determined using enzyme linked immunosorbent assays (ELISA). Briefly, a monoclonal antibody specific to the MMAF portion of the antibody-drug conjugate was used to capture the SGN-CD19A antibody from plasma. Bound SGN-CD19A was detected by a biotin conjugated anti-ID19A detection antibody followed by addition of a polymer horseradish peroxidase conjugated to streptavidin (poly HRP-SA). For detection TMB (3,3',5,5' tetramethylbenzidine) was used which was converted from colorless TMB to a colored TMB derivative.

Unbound CD19 receptor protein on blasts - Blasts were isolated from cryopreserved patient blood and incubated labeled hBU12 antibody (hBU12-AF488). After incubation the cells were assayed by flow cytometry for its fluorescence intensity expressed as mean fluorescence intensity (MFI).

SGN-CD19A bound to blast cells - Blast cells were isolated from patient blood and incubated with a labeled antibody specific for MMAF (SG15.22-AF647). After washing to remove unbound labeled antibody, cells were assayed by flow cytometry for its fluorescence intensity expressed as MFI.

**Table 2 PK/PD sample collection timepoints**

| Cycle | Study Day | Time | Window | Relative Time | Adult PK | Pediatric PK | PD |
|---|---|---|---|---|---|---|---|
| | | Pre-dose | within 24 hr | START of infusion | x | x | x |
| 1^{c} | Day 1 | End of infusion | within 15 min | END of infusion | x | x | x |
| | | 2 h | +/- 15 min | END of infusion | x | x | |
| | | 4 h | +/- 15 min | END of infusion | x | x | |
| | | 8 h | +/- 15 min | END of infusion | x | x | x |
| | Day 2 | 24 h | +/- 4 hr | START of infusion | x | x | |
| | Day 4 | 72 h | +/- 4 hr | START of infusion | x | x | x |
| | Day 8 | Pre-dose | within 4 hr | START of infusion | x | x | x |
| | | End of infusion | within 15 min | END of infusion | x | x | |
| | | 2 h | +/- 15 min | END of infusion | x | x | |
| | | 4 h | +/- 15 min | END of infusion | x | x | |
| | | 8 h | +/- 15 min | END of infusion | x | x | |
| | Day 9 | 24 h | +/- 4 hr | START of infusion | x | x | |
| | Day 11 | 72 h | +/- 4 hr | START of infusion | x | x | |
| | Day 15 | 168 h | +/- 24 hr | START of infusion | x | x | |
| | Day 22^{a} | 336 h | +/- 24 hr | START of infusion | x | x | x |
| | Day 1 | Pre-dose | within 4 hr | START of infusion | x | x | x |
| 2 and 4 | | End of infusion | within 15 min | END of infusion | x | x | |
| | Day 8 | Pre-dose | within 4 hr | START of infusion | x | x | |
| | | End of infusion | within 15 min | END of infusion | x | x | |
| | | 2 h | +/- 15 min | END of infusion | x | x | |
| | | 4 h | +/- 15 min | END of infusion | x | x | |
| | | 8 h | +/- 15 min | END of infusion | x | x | |
| | Day 9 | 24 h | +/- 4 hr | START of infusion | x | x | |
| | Day 11 | 72 h | +/- 4 hr | START of infusion | x | x | x^{b} |
| | Day 15 | 168 h | +/- 24 hr | START of infusion | x | x | |
| | Day 22^{a} | 336 h | +/- 24 hr | START of infusion | x | x | |
| Cycle 3 and all other cycles | Day 1 | Pre-dose | within 4 hr | START of infusion | x | x | x |
| | | End of infusion | within 15 min | END of infusion | x | x | |
| | Day 8 | Pre-dose | within 4 hr | START of infusion | x | | |
| | | End of infusion | within 15 min | END of infusion | x | | |
| | Day 15 | 168 h | +/- 24 hr | START of infusion | x | | |
| | Day 22^{a} | 336 h | +/- 24 hr | START of infusion | x | x | |
| | EOT | | | | x | x | x |

| | | | | | | | |
|---|---|---|---|---|---|---|---|
| ^{a} Sample obtained only for patients not continuing on treatment or not starting the next cycle on Day 22. ^{b} Cycle 2 only. ^{c} This schedule should be followed for retreatment cycle 1 | | | | | | | |

### Measurement of disease burden

Patient disease was monitored by bone marrow aspiration and/or biopsy at indicated times. A pathologist reviewed bone marrow slides from patients and assessed for percentage of bone marrow cells that were malignant blasts, as well as the total amount of space in the bone marrow that was taken up by cells (cellularity). Disease burden was a product of the cellularity and the percentage of the bone marrow that was replaced by malignant blasts.

### Results

### PK in ALL and lymphoma

SGN-CD19A was administered to ALL patients at levels up to 2.0 mg/kg on days 1 and 8 of a 21 day cycle. Plasma levels of SGN-CD19A were measured per protocol-defined schedule (Table 2). Results are shown in Figure 2A. Following iv infusion administration, the levels of plasma SGN-CD19A generally reached Cmax at the end of infusion and decreased in a multiexponential manner. The PK profiles showed a more rapid decline of the SGN-CD19A plasma concentration at lower dose levels, indicating non-linear PK. In contrast, when SGN-CD19A was administered to lymphoma patients the PK profiles are similar among different dose levels, as shown in Figure 2B

### Semi-mechanistic integrated PK-PD model

A semi-mechanistic integrated PK-PD model was developed based on the mechanism of action of SGN-CD19A and the clinical PK and PD data from the ongoing first-in-human dose-escalation study in adult and pediatric patients with relapsed or refractory B cell leukemia or highly aggressive B-cell lymphoma (CT.gov NCT01786096). Plasma SGN-CD19A ADC concentration-time profiles were described by a two-compartment PK model with both linear and nonlinear clearance pathways. The nonlinear clearance was modeled as target-mediated disposition through binding to cell membrane CD19 and internalization of the complex. Antitumor effects in the form of reduced bone marrow disease burden were modeled using a cell-kill PK-PD model driven by the internalized cys-mcMMAF concentrations. Both PK and PD data were fitted simultaneously using NONMEM® version 7.1.2. At low levels of administration, SGN-CD19A exhibited a biphasic PK curve, indicating rapid clearance of the drug. As the dose of SGN-CD19A increased the PK curve became monophasic indicating slower clearance of the drug. SGN-CD19A clearance occurred through two mechanisms: clearance through whole body catabolism and clearance through uptake by tumor cells. Over time SGN-CD19A was taken up by ALL tumor cells, killing the tumor cells and reducing the tumor burden. Thus, over time, the rate of clearance through tumor uptake decreased and less SGN-CD19A was needed to kill tumor cells.

### Model diagnostics

To determine the goodness of model fit, model-predicted values were compared to values observed from patient samples. Results are shown in figures 3A-3D. In Figure 3A observed plasma SGN-CD19A concentrations were plotted against the predicted values from the model. Linear regression analysis was performed and compared to a unity line. In Figure 3B levels of observed unbound CD 19 MFI on blast cells were plotted against the predicted values from the model. Linear regression analysis was performed and compared to a unity line. In Figure 3C levels of observed MAFI for SGN-CD19A bound to blast cells were plotted against the predicted values from the model. Linear regression analysis was performed and compared to a unity line. In Figure 3D levels of observed percent blast cells in bone marrow were plotted against the predicted values from the model. Linear regression analysis was performed and compared to a unity line. For all of the comparisons, the linear regression and unity lines were nearly identical, indicating that the observed and predicted values were similar and the model described the observed data well .

Figures 4A-4D show observed and predicted values for a single ALL patient over a period of time. In all graphs, 0.3 mg/kg SGN-CD19A was administered on days 1 and 8 of cycles 1-4; 1.0 mg/kg SGN-CD19A was administered on days 1 and 8 of cycles 5-8; and 1.3 mg/kg was administered on days 1 and 8 of cycle 9. In Figure 4A observed plasma SGN-CD19A (ADC) concentrations were plotted against time and then overlaid with the predicted values from the model. In Figure 4B levels of observed unbound CD19 MFI on blast cells were plotted against time and then overlaid with the predicted values from the model. In Figure 4C levels of observed MFI for SGN-CD19A bound to blast cells were plotted against time and then overlaid with the predicted values from the model. In Figure 4D levels of observed percent blast cells in bone marrow were plotted against time and then overlaid with the predicted values from the model. For all of these comparisons, the pattern predicted by the model matched the observed values in patient samples well.

### Simulation of CD19 occupancy based on model

Model simulations were conducted for alternative doses and schedules, including more frequent dose or loading dose regimens followed by maintenance dose regimens, and for ALL patients with different baseline disease characteristics.

The first simulation was for an ALL patient with high baseline CD19 expression and 100% bone marrow blasts and hypercellularity in bone marrow. SGN-CD19A was administered on a fixed dosage schedules: 1.6 mg/kg on days 1 and 8 of a 21 day cycle; 2.2 mg/kg every two weeks; or 3 mg/kg every three weeks. The model was used to predict the percentage of CD19 on blast cells that were bound to SGN-CD19A (CD19 occupancy). High target saturation was assumed to occur if 90% of the CD19 proteins on blasts were occupied. Figure 5A provides the results for cancer that is sensitive to treatment, while Figure 5B provides the results for a cancer that is insensitive to treatment. Simulation results suggest that at dose intensity of ∼1 mg/kg/week, all dosing schedules appear to achieve high and comparable target occupancy by SGN-CD19A after Cycle 1; however, target saturation could drop to lower level in Cycle 1.

A simulation was run for an ALL patient with high baseline CD19 expression and 10% bone marrow blasts and hypercellularity. SGN-CD19A was administered on a fixed dosage schedules: 1.6 mg/kg on days 1 and 7 of a 21 day cycle; 2.2 mg/kg every two weeks; or 3 mg/kg every three weeks. The model was used to predict the percentage of CD19 on blast cells that were bound to SGN-CD19A (CD19 occupancy). High target saturation was assumed to occur if 90% of the CD19 proteins on blasts were occupied. Figure 6A provides the results for cancer that is sensitive to treatment, while Figure 6B provides the results for a cancer that is insensitive to treatment. Simulation results suggest that at dose intensity of ∼1 mg/kg/week, all dosing schedules appear to achieve high and comparable target occupancy by SGN-CD19A.

A simulation was run for an ALL patient with low baseline CD19 expression and 100% bone marrow blasts with blast hypercellularity. As above, SGN-CD19A was administered on a fixed dosage schedules: 1.6 mg/kg on days 1 and 7 of a 21 day cycle; 2.2 mg/kg every two weeks; or 3 mg/kg every three weeks. The model was used to predict the percentage of CD 19 on blast cells that were bound to SGN-CD19A (CD19 occupancy). High target saturation was assumed to occur if 90% of the CD19 proteins on blasts were occupied. Figure 7A provides the results for cancer that is sensitive to treatment, while Figure 7B provides the results for a cancer that is insensitive to treatment. Simulation results suggest that at dose intensity of ∼1 mg/kg/week, all dosing schedules appear to achieve high and comparable target occupancy by SGN-CD19A.

The remaining simulations tested more frequent doses or loading doses of SGN-CD19A followed by less frequent and/or lower maintenance doses. A simulation was run for an ALL patient with high baseline CD19 expression and 100% bone marrow blasts and hypercellularity. The dose scenario used 2 mg/kg dose given on days 1 and 8 of a twenty-one day cycle for the entire test period. The other doses started with 2 mg/kg given on days 1 and 8 of a twenty-one day cycle, followed by 2, 1 or 0.5 mg/kg given every three weeks. Figure 8A provides the results for cancer that is sensitive to treatment, while Figure 8B provides the results for a cancer that is less sensitive to treatment. Simulation results suggest that when given every three weeks a dose of 2 mg/kg is needed to achieve high CD19 occupancy by SGN-CD19A during maintenance for sensitive disease while more than 2 mg/kg is needed for less sensitive disease.

A loading dose simulation was run for an ALL patient with high baseline CD19 expression and 100% bone marrow blasts and hypercellularity. This loading dose scenario used 5 mg/kg given on days 1 of a twenty-one day cycle for the entire test period. The other doses started with 5 mg/kg given on days 1 of a twenty-one day cycle, followed by 3, 2, or 1 mg/kg given every three weeks. Figure 9A provides the results for cancer that is sensitive to treatment, while Figure 9B provides the results for a cancer that is less sensitive to treatment. Simulation results suggest that when given every three weeks a dose of 2 mg/kg is needed to achieve high CD 19 occupancy by SGN-CD19A during maintenance for sensitive disease while 3 mg/kg is needed for less sensitive disease.

A loading dose simulation was run for an ALL patient with low baseline CD19 expression and 100% bone marrow blasts with blast hypercellularity. This dose scenario used 2 mg/kg given on days 1 and 8 of a twenty-one day cycle for the entire test period. The other doses started with 2 mg/kg given on days 1 and 8 of a twenty-one day cycle, followed by 2, 1 or 0.5 mg/kg given every three weeks. Figure 10A provides the results for cancer that is sensitive to treatment, while Figure 10B provides the results for a cancer that is less sensitive to treatment. Simulation results suggest that when given every three weeks a dose of 2 mg/kg is needed to achieve high CD19 occupancy by SGN-CD19A during maintenance for sensitive and less sensitive diseases.

A loading dose simulation was run for an ALL patient with low baseline CD19 expression and 100% bone marrow blasts with blast hypercellularity. This loading dose scenario used 5 mg/kg given on days 1 of a twenty-one day cycle for the entire test period. The other doses started with 5 mg/kg given on days 1 of a twenty-one day cycle, followed by 3, 2, or 1 mg/kg given every three weeks. Figure 11A provides the results for cancer that is sensitive to treatment, while Figure 11B provides the results for a cancer that is less sensitive to treatment. Simulation results suggest that when given every three weeks a dose of 2 mg/kg is needed to achieve high CD19 occupancy by SGN-CD19A during maintenance for sensitive and less sensitive diseases.

It is understood that the examples and embodiments described herein are for illustrative purposes only.

## Claims

1. A CD19-antibody drug conjugate (CD19-ADC) for use in treating acute lymphoblastic leukemia, wherein the CD19-ADC is a humanized BU12 antibody conjugated to an mcMMAF molecule wherein the humanized BU12 antibody has a light chain variable region of SEQ ID NO:1 and a heavy chain variable region of SEQ ID NO:2, the method comprising the steps of
a) administering a loading dose of the CD19-ADC, followed by
b) administering a maintenance dose of the CD19-ADC, wherein the maintenance dose of the CD19-ADC is 2.0 mg/kg body weight and is administered every three weeks.

2. The CD19-antibody drug conjugate (CD19-ADC) for use of claim 1, wherein the loading dose is 2.0 mg/kg body weight and is administered on days one and eight of a three week cycle.

3. The CD19-antibody drug conjugate (CD19-ADC) for use of claim 2, wherein the loading dose is administered for one three week cycle.

4. The CD19-antibody drug conjugate (CD19-ADC) for use of claim 2, wherein the loading dose is administered for two three week cycles.

5. The CD19-antibody drug conjugate (CD19-ADC) for use of claim 2, wherein the loading dose is administered for between one and four three week cycles.

6. The CD19-antibody drug conjugate (CD19-ADC) for use of claim 2, wherein the maintenance dose is administered for multiple three week cycles.

7. The CD19-antibody drug conjugate (CD19-ADC) for use of claim 2, wherein the maintenance dose is administered for between one and ten three week cycles.

## Patentansprüche

1. CD19-Antikörper-Arzneimittelkonjugat (CD19-ADC) zur Verwendung bei der Behandlung von akuter lymphoblastischer Leukämie, wobei das CD19-ADC ein humanisierter BU12-Antikörper ist, der mit einem mcMMAF-Molekül konjugiert ist, wobei der humanisierte BU12-Antikörper einen variablen Bereich der leichten Kette von SEQ ID NO: 1 und einen variablen Bereich der schweren Kette von SEQ ID NO: 2 aufweist, das Verfahren folgende Schritte umfassend:
a) Verabreichen einer Anfangsdosis des CD19-ADC, mit anschließendem
b) Verabreichen einer Erhaltungsdosis des CD19-ADC, wobei die Erhaltungsdosis des CD19-ADC 2,0 mg/kg Körpergewicht beträgt und alle drei Wochen verabreicht wird.

2. CD19-Antikörper-Arzneimittelkonjugat (CD19-ADC) zur Anwendung nach Anspruch 1, wobei die Anfangsdosis 2,0 mg/kg Körpergewicht beträgt und jeweils am ersten und achten Tag eines Drei-Wochen-Zyklus verabreicht wird.

3. CD19-Antikörper-Arzneimittelkonjugat (CD19-ADC) nach Anspruch 2, wobei die Anfangsdosis über einen Drei-Wochen-Zyklus verabreicht wird.

4. CD19-Antikörper-Arzneimittelkonjugat (CD19-ADC) nach Anspruch 2, wobei die Anfangsdosis über zwei Zyklen verabreicht wird.

5. CD19-Antikörper-Arzneimittelkonjugat (CD19-ADC) nach Anspruch 2, wobei die Anfangsdosis über einen bis vier Drei-Wochen-Zyklen verabreicht wird.

6. CD19-Antikörper-Arzneimittelkonjugat (CD19-ADC) nach Anspruch 2, wobei die Erhaltungsdosis über mehrere Drei-Wochen-Zyklen verabreicht wird.

7. CD19-Antikörper-Arzneimittelkonjugat (CD19-ADC) nach Anspruch 2, wobei die Erhaltungsdosis über einen bis zehn Drei-Wochen-Zyklen verabreicht wird.

## Revendications

1. Conjugué médicament-anticorps anti-CD19 (CD19-ADC) destiné à être utilisé pour le traitement de la leucémie lymphoblastique aiguë, dans lequel le CD19-ADC est un anticorps BU12 humanisé conjugué à une molécule mcMMAF, dans lequel l'anticorps BU12 humanisé comporte une région variable de chaîne légère de SEQ ID N° : 1 et une région variable de chaîne lourde de SEQ ID N° : 2, le procédé comprenant les étapes
a) d'administration d'une dose de charge du CD19-ADC, suivie de
b) l'administration d'une dose d'entretien du CD19-ADC, dans lequel la dose d'entretien du CD19-ADC est de 2,0 mg/kg de poids corporel et est administrée toutes les trois semaines.

2. Conjugué de médicament CD19-anticorps (CD19-ADC) destiné à être utilisé selon la revendication 1, dans lequel la dose de charge est de 2,0 mg/kg de poids corporel et est administrée au premier et au huitième jour d'un cycle de trois semaines.

3. Conjugué de médicament CD19-anticorps (CD19-ADC) destiné à être utilisé selon la revendication 2, dans lequel la dose de charge est administrée pendant un cycle de trois semaines.

4. Conjugué de médicament CD19-anticorps (CD19-ADC) destiné à être utilisé selon la revendication 2, dans lequel la dose de charge est administrée pendant deux cycles de trois semaines.

5. Conjugué de médicament CD19-anticorps (CD19-ADC) destiné à être utilisé selon la revendication 2, dans lequel la dose de charge est administrée pendant un à quatre cycles de trois semaines.

6. Conjugué médicament-anticorps anti-CD19 (CD19-ADC) destiné à être utilisé selon la revendication 2, dans lequel la dose d'entretien est administrée pendant plusieurs cycles de trois semaines.

7. Conjugué médicament-anticorps anti-CD19 (CD19-ADC) destiné à être utilisé selon la revendication 2, dans lequel la dose d'entretien est administrée pendant un à dix cycles de trois semaines.


### SEQUENCE LISTING

<110> SEATTLE GENETICS, INC.
<120> OPTIMAL DOSING OF A CD19-ANTIBODY DRUG CONJUGATE
<130> 0019-00411PC
<150> US 61/976,790
   <151> 2014-04-08
<160> 2
<170> PatentIn version 3.5
<210> 1
   <211> 107
   <212> PRT
   <213> Artificial
<220>
   <223> hBU12 light chain variable region
<400> 1
   Glu Ile Val Leu Thr Gln Ser Pro Ala Thr Leu Ser Leu Ser Pro Gly
   1               5                   10                  15
   Glu Arg Ala Thr Leu Ser Cys Ser Ala Ser Ser Ser Val Ser Tyr Met
               20                  25                  30
   His Trp Tyr Gln Gln Lys Pro Gly Gln Ala Pro Arg Leu Leu Ile Tyr
           35                  40                  45
   Asp Thr Ser Lys Leu Ala Ser Gly Ile Pro Ala Arg Phe Ser Gly Ser
       50                  55                  60
   Gly Ser Gly Thr Asp Phe Thr Leu Thr Ile Ser Ser Leu Glu Pro Glu
   65                  70                  75                  80
   Asp Val Ala Val Tyr Tyr Cys Phe Gln Gly Ser Val Tyr Pro Phe Thr
                   85                  90                  95
   Phe Gly Gln Gly Thr Lys Leu Glu Ile Lys Arg
               100                 105
<210> 2
   <211> 120
   <212> PRT
   <213> Artificial
<220>
   <223> hBU12 heavy chain variable region
<400> 2
   Gln Val Gln Leu Gln Glu Ser Gly Pro Gly Leu Val Lys Pro Ser Gln
   1               5                   10                  15
   Thr Leu Ser Leu Thr Cys Thr Val Ser Gly Gly Ser Ile Ser Thr Ser
               20                  25                  30
   Gly Met Gly Val Gly Trp Ile Arg Gln His Pro Gly Lys Gly Leu Glu
           35                  40                  45
   Trp Ile Gly His Ile Trp Trp Asp Asp Asp Lys Arg Tyr Asn Pro Ala
       50                  55                  60
   Leu Lys Ser Arg Val Thr Ile Ser Val Asp Thr Ser Lys Asn Gln Phe
   65                  70                  75                  80
   Ser Leu Lys Leu Ser Ser Val Thr Ala Ala Asp Thr Ala Val Tyr Tyr
                   85                  90                  95
   Cys Ala Arg Met Glu Leu Trp Ser Tyr Tyr Phe Asp Tyr Trp Gly Gln
               100                 105                 110
   Gly Thr Leu Val Thr Val Ser Ser
           115                 120